# EUROPEAN PATENT APPLICATION

(11) **EP 4 001 301 A1**
(43) Date of publication of application: **25.05.2022**
(21) Application number: 20207536.2
(22) Date of filing: 13.11.2020
(51) Int. Cl.: C07K 14/47, A61K 38/00, C07K 14/31

(54) **BINDING PROTEIN FOR COMPLEMENT FACTOR H (CFH)**

(71) Applicant: NAVIGO PROTEINS GMBH, 06120 Halle/Saale (DE)
(72) Inventor: Kahl, Mathias, 06120 Halle/Saale (DE); Fiedler, Erik, 06120 Halle/Saale (DE); Lotze, Jonathan, 06120 Halle/Saale (DE)
(74) Representative: Haucke, Kerstin

(57) **Abstract**

The present invention relates to novel proteins that specifically bind to complement factor H (CFH). The present invention relates to the field of the purification of CFH. The novel proteins of the present invention are advanced and powerful tools because they allow precise capturing of CFH in affinity chromatography.

SEQUENCE LISTING

<110>  Navigo Proteins GmbH

<120>  Binding proteins for Complement Factor H (CFH)

<130>  NP52 EP1

<160>  28

<170>  PatentIn version 3.5

<210>  1
<211>  58
<212>  PRT
<213>  Artificial Sequence

<220>
<223>  CFH binding protein 209202

<400>  1

Asn Ala Ala Asp Leu Asp Glu Ala Gln Ile Ser Ala Ile Phe Glu Ile
1               5                   10                  15

Met Asp Leu Pro Asn Leu Thr Trp Gln Gln Gln Glu Trp Phe Ile Asp
            20                  25                  30

Ser Leu Ser Asp Asp Pro Ser Val Ser Lys Glu Val Leu Gly Glu Ala
        35                  40                  45

Gln Lys Leu Asn Asp Ser Gln Ala Pro Lys
    50                  55

<210>  2
<211>  58
<212>  PRT
<213>  Artificial Sequence

<220>
<223>  CFH binding protein 209264

<400>  2

Ile Ala Ala Lys Phe Asp Arg Ile Gln Ser Ile Ala Asp Glu Val Ile
1               5                   10                  15

Leu Ile Leu Pro Asn Leu Thr Glu Glu Gln Arg Asn Ala Phe Arg Gln
            20                  25                  30

Ser Leu Ser Asp Asp Pro Ser Val Ser Glu Glu Val Leu Tyr Glu Ala
        35                  40                  45

Leu Tyr Leu Asn Tyr Ser Gln Ala Pro Lys
    50                  55

<210> 3
<211> 58
<212> PRT
<213> Artificial Sequence

<220>
<223> CFH binding protein 209274

<400> 3

Ile Ala Ala Arg Phe Asp Lys Ala Gln Arg Ala Ala Glu Leu Glu Ile
1               5                   10                  15

Lys Ala Leu Pro Asn Leu Thr Tyr Glu Gln Arg Lys Ala Phe Arg Val
            20                  25                  30

Ser Leu Tyr Asp Asp Pro Ser Val Ser Leu Glu Val Leu Gly Glu Ala
        35                  40                  45

Gln Lys Leu Asn Asp Ser Gln Ala Pro Lys
    50                  55

<210> 4
<211> 58
<212> PRT
<213> Artificial Sequence

<220>
<223> CFH binding protein 209275

<400> 4

Ile Ala Ala Arg Phe Asp His Ala Gln Trp Ala Ala Glu Leu Glu Ile
1               5                   10                  15

Lys Glu Leu Pro Asn Leu Thr Tyr Glu Gln Arg Lys Ala Phe Arg Ile
            20                  25                  30

Ser Leu Tyr Asp Asp Pro Ser Val Ser Leu Glu Val Leu Gly Glu Ala
        35                  40                  45

Gln Lys Leu Asn Asp Ser Gln Ala Pro Lys
    50                  55

<210> 5
<211> 58
<212> PRT
<213> Artificial Sequence

<220>
<223> CFH binding protein 209447

<400> 5

Asn Ala Ala Glu Ala Asp Asp Ala Gln Val Ser Ala Ala Met Glu Ile
1               5                   10                  15

Ile Met Leu Pro Asn Leu Thr Leu Glu Gln Ala His Ser Phe Ile Glu
            20                  25                  30

Ser Leu Tyr Arg Asp Pro Ser Val Ser Lys Glu Val Leu Gly Glu Ala
        35                  40                  45

Gln Lys Leu Asn Asp Ser Gln Ala Pro Lys
    50                  55

<210>  6
<211>  58
<212>  PRT
<213>  Artificial Sequence

<220>
<223>  CFH binding protein 209466

<400>  6

Asn Ala Ala Asp Lys Asp Phe Ala Gln Glu Ser Ala Glu Tyr Glu Ile
1               5                   10                  15

Leu Met Leu Pro Asn Leu Thr Leu Asp Gln Ala Tyr Ser Phe Ile Leu
            20                  25                  30

Ser Leu Lys Ser Asp Pro Ser Val Ser Lys Glu Val Leu Gly Glu Ala
        35                  40                  45

Gln Lys Leu Asn Asp Ser Gln Ala Pro Lys
    50                  55

<210>  7
<211>  116
<212>  PRT
<213>  Artificial Sequence

<220>
<223>  CFH binding protein 208851

<400>  7

Ile Ala Ala Lys Phe Asp Glu Ala Gln Trp Ala Ala Phe Tyr Glu Ile
1               5                   10                  15

Val His Leu Pro Asn Leu Thr Glu Glu Gln Arg Glu Ala Phe Ile Gln
            20                  25                  30

Ser Leu Trp Asp Asp Pro Gly Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Ala Gln Arg Asn His Phe Ile Gln Arg Leu Arg Asp Asp Pro
                85                  90                  95

Ser Val Ser Leu Val Val Leu Gly Gln Ala Gln Lys Leu Asn Asp Ser
            100                 105                 110

Gln Ala Pro Lys
        115

<210>  8
<211>  116
<212>  PRT
<213>  Artificial Sequence

<220>
<223>  CFH binding protein 208854

<400>  8

Ile Ala Ala Lys Phe Asp Glu Ala Gln Gln Lys Ala Phe Tyr Trp Ile
1               5                   10                  15

Leu His Leu Arg Asn Leu Thr Glu Glu Gln Arg Asn Ala Phe Ile Gln
            20                  25                  30

Ser Leu Arg Asp Asp Pro Ser Val Ser Thr Glu Val Leu Gly Glu Ala
        35                  40                  45

Leu Lys Leu Asn Gln Ser Gln Ala Pro Lys Ile Ala Ala Lys Phe Asp
    50                  55                  60

Glu Ala Gln Trp Ala Ala Phe Tyr Glu Ile Val His Leu Pro Asn Leu
65                  70                  75                  80

Thr Glu Glu Gln Arg Glu Ala Phe Ile Gln Ser Leu Trp Asp Asp Pro
                85                  90                  95

Ser Val Ser Leu Glu Val Leu Gly Glu Ala Gln Lys Leu Asn Asp Ser
            100                 105                 110

Gln Ala Pro Lys
        115

<210>  9
<211>  116
<212>  PRT
<213>  Artificial Sequence

<220>
<223>  CFH binding protein 209354

<400>  9

Ile Ala Ala Lys Phe Asp Glu Ala Gln Trp Ala Ala Phe Tyr Glu Ile
1               5                   10                  15

Val His Leu Pro Asn Leu Thr Glu Glu Gln Arg Glu Ala Phe Ile Gln
            20                  25                  30

Ser Leu Trp Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu His Gln Arg Asn His Phe Ile Gln Lys Leu Arg Asp Asp Pro
                85                  90                  95

Ser Val Ser Leu Gln Val Leu Gly His Ala Gln Arg Leu Asn Asp Ser
            100                 105                 110

Gln Ala Pro Lys
        115


<210>  10
<211>  116
<212>  PRT
<213>  Artificial Sequence

<220>
<223>  CFH binding protein 209372

<400>  10

Ile Ala Ala Lys Phe Asp Glu Ala Gln Trp Ala Ala Phe Tyr Glu Ile
1               5                   10                  15

Val His Leu Pro Asn Leu Thr Glu Glu Gln Arg Glu Ala Phe Ile Gln
            20                  25                  30

Ser Leu Trp Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Gln Gln Arg Asn Tyr Phe Ile Gln Arg Leu Arg Asp Asp Pro
                85                  90                  95

Ser Val Ser Leu Ala Val Leu Gly His Ala Gln Lys Leu Asn Asp Ser
            100                 105                 110

Gln Ala Pro Lys
        115

<210>  11
<211>  116
<212>  PRT
<213>  Artificial Sequence

<220>
<223>  CFH binding protein 209429

<400>  11

Ile Ala Ala Lys Phe Asp Glu Ala Gln Gln Arg Ala Phe Tyr Trp Ile
1               5                   10                  15

Leu His Leu Arg Asn Leu Thr Glu Glu Gln Arg Asn Ala Phe Ile Gln
            20                  25                  30

Ser Leu Arg Asp Asp Pro Ser Val Ser Lys Glu Val Leu Gly Glu Ala
        35                  40                  45

Tyr Lys Leu Asn Lys Ser Gln Ala Pro Lys Ile Ala Ala Lys Phe Asp
    50                  55                  60

Glu Ala Gln Thr Ala Ala Phe Trp Glu Ile Trp His Leu Pro Asn Leu
65                  70                  75                  80

Thr Glu Glu Gln Arg Val Ala Phe Ile His Ser Leu Gln Asp Asp Pro
                85                  90                  95

Ser Val Ser Leu Glu Val Leu Gly Glu Ala Gln Lys Leu Asn Asp Ser
            100                 105                 110

Gln Ala Pro Lys
        115

<210>  12
<211>  116
<212>  PRT
<213>  Artificial Sequence

<220>
<223>  CFH binding protein 209434

<400>  12

Ile Ala Ala Lys Phe Asp Glu Ala Gln Gln Arg Ala Phe Tyr Trp Ile
1               5                   10                  15

Leu His Leu Arg Asn Leu Thr Glu Glu Gln Arg Asn Ala Phe Ile Gln
            20                  25                  30

Ser Leu Arg Asp Asp Pro Ser Val Ser Lys Glu Val Leu Gly Glu Ala
        35                  40                  45

Tyr Lys Leu Asn Val Ser Gln Ala Pro Lys Ile Ala Ala Lys Phe Asp
    50                  55                  60

Glu Ala Gln Val Ala Ala Phe Trp Glu Ile Trp His Leu Pro Asn Leu
65                  70                  75                  80

Thr Glu Glu Gln Arg Val Ala Phe Ile His Ser Leu Gln Asp Asp Gln
                85                  90                  95

Ser Val Ser Leu Glu Val Leu Gly Glu Ala Gln Lys Leu Asn Asp Ser
            100                 105                 110

Gln Ala Pro Lys
        115


<210>  13
<211>  116
<212>  PRT
<213>  Artificial Sequence

<220>
<223>  CFH binding protein 209621

<400>  13

Ile Ala Ala Lys Phe Asp Glu Ala Gln Thr Ala Ala Phe Trp Glu Ile
1               5                   10                  15

Glu His Leu Pro Asn Leu Thr Glu Glu Gln Arg Trp Ala Phe Ile Trp
            20                  25                  30

Ser Leu Ala Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Trp Gln Arg Asn Tyr Phe Ile Gln Lys Leu Arg Asp Asp Pro
                85                  90                  95

Ser Val Ser Leu Trp Val Leu Gly Gln Ala Gln Ile Leu Asn Asp Ser
            100                 105                 110

Gln Ala Pro Lys
        115

<210>  14
<211>  116
<212>  PRT
<213>  Artificial Sequence

<220>
<223>  CFH binding protien 209826

<400>  14

Ile Ala Ala Lys Phe Asp Glu Ala Gln Glu Ala Ala Phe Trp Glu Ile
1               5                   10                  15

Gln His Leu Pro Asn Leu Thr Glu Glu Gln Arg Trp Ala Phe Ile Trp
            20                  25                  30

Ser Leu Ala Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Tyr Gln Arg Asn Leu Phe Ile Gln Arg Leu Arg Asp Asp Pro
                85                  90                  95

Ser Val Ser Leu Trp Val Leu Gly Gln Ala Gln Ile Leu Asn Asp Ser
            100                 105                 110

Gln Ala Pro Lys
        115

<210>  15
<211>  116
<212>  PRT
<213>  Artificial Sequence

<220>
<223>  CFH binding protein 209827

<400>  15

Ile Ala Ala Lys Phe Asp Glu Ala Gln Glu Ala Ala Phe Trp Glu Ile
1               5                   10                  15

Gln His Leu Pro Asn Leu Thr Glu Glu Gln Arg Trp Ala Phe Ile Trp
            20                  25                  30

Ser Leu Ala Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Trp Gln Arg Asn Tyr Phe Ile Gln Lys Leu Arg Asp Asp Pro
                85                  90                  95

Ser Val Ser Leu Trp Val Leu Gly Gln Ala Gln Ile Leu Asn Asp Ser
            100                 105                 110

Gln Ala Pro Lys
        115


<210>  16
<211>  116
<212>  PRT
<213>  Artificial Sequence

<220>
<223>  CFH binding protein 209831

<400>  16

Ile Ala Ala Lys Phe Asp Glu Ala Gln Trp Ala Ala Phe Tyr Glu Ile
1               5                   10                  15

Val His Leu Pro Asn Leu Thr Glu Glu Gln Arg Glu Ala Phe Ile Gln
            20                  25                  30

Ser Leu Trp Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Arg Gln Arg Asn Trp Phe Ile Gln Arg Leu Arg Asp Asp Pro
                85                  90                  95

Ser Val Ser Leu Trp Val Leu Gly Arg Ala Gln Arg Leu Asn Asp Ser
            100                 105                 110

Gln Ala Pro Lys
        115

<210>  17
<211>  116
<212>  PRT
<213>  Artificial Sequence

<220>
<223>  CFH binding protein 209833

<400>  17

Ile Ala Ala Lys Phe Asp Glu Ala Gln Glu Ala Ala Phe Trp Glu Ile
1               5                   10                  15

Gln His Leu Pro Asn Leu Thr Glu Glu Gln Arg Trp Ala Phe Ile Trp
            20                  25                  30

Ser Leu Ala Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu His Gln Arg Asn Leu Phe Ile Gln Arg Leu Arg Asp Asp Pro
                85                  90                  95

Ser Val Ser Leu Trp Val Leu Gly Gln Ala Gln Ile Leu Asn Asp Ser
            100                 105                 110

Gln Ala Pro Lys
        115

<210>  18
<211>  116
<212>  PRT
<213>  Artificial Sequence

<220>
<223>  CFH binding protein 209835

<400>  18

Ile Ala Ala Lys Phe Asp Glu Ala Gln Trp Ala Ala Phe Tyr Glu Ile
1               5                   10                  15

Val His Leu Pro Asn Leu Thr Glu Glu Gln Arg Glu Ala Phe Ile Gln
            20                  25                  30

Ser Leu Trp Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Arg Gln Arg Asn Trp Phe Ile Gln Gln Leu Arg Asp Asp Pro
                85                  90                  95

Ser Val Ser Leu Thr Val Leu Gly Arg Ala Gln Arg Leu Asn Asp Ser
            100                 105                 110

Gln Ala Pro Lys
        115


<210>  19
<211>  116
<212>  PRT
<213>  Artificial Sequence

<220>
<223>  CFH binding protien 209860

<400>  19

Ile Ala Ala Lys Phe Asp Glu Ala Gln Trp Ala Ala Phe Glu Glu Ile
1               5                   10                  15

Ala His Leu Pro Asn Leu Thr Glu Glu Gln Arg Glu Ala Phe Ile Gln
            20                  25                  30

Ser Leu Trp Asp Asp Pro Ser Val Ser Leu Glu Val Leu Gly Glu Ala
        35                  40                  45

Gln Lys Leu Asn Asp Ser Gln Ala Pro Lys Ile Ala Ala Lys Phe Asp
    50                  55                  60

Glu Ala Gln Gln Ala Ala Phe Tyr Glu Ile Leu His Leu Pro Asn Leu
65                  70                  75                  80

Thr Glu Arg Gln Arg Asn Trp Phe Ile Gln Arg Leu Arg Asp Asp Pro
                85                  90                  95

Ser Val Ser Leu Trp Val Leu Gly Arg Ala Gln Arg Leu Asn Asp Ser
            100                 105                 110

Gln Ala Pro Lys
        115

<210>  20
<211>  116
<212>  PRT
<213>  Artificial Sequence

<220>
<223>  CFH binding protein 209864

<400>  20

Ile Ala Ala Lys Phe Asp Glu Ala Gln Gln Ala Ala Phe Tyr Glu Ile
1               5                   10                  15

Leu His Leu Pro Asn Leu Thr Glu Leu Gln Arg Asn His Phe Ile Gln
            20                  25                  30

Thr Leu Arg Asp Asp Pro Ser Val Ser Leu Trp Val Leu Gly Leu Ala
        35                  40                  45

Gln Leu Leu Asn Asp Ser Gln Ala Pro Lys Ile Ala Ala Lys Phe Asp
    50                  55                  60

Glu Ala Gln Gln His Ala Phe Tyr Thr Ile Leu His Leu Glu Asn Leu
65                  70                  75                  80

Thr Glu Glu Gln Arg Asn Ala Phe Ile Gln Ser Leu Arg Asp Asp Pro
                85                  90                  95

Ser Val Ser Leu Glu Val Leu Gly Glu Ala Ile Lys Leu Asn Ile Ser
            100                 105                 110

Gln Ala Pro Lys
        115

<210>  21
<211>  116
<212>  PRT
<213>  Artificial Sequence

<220>
<223>  CFH binding protein 209920

<400>  21

Ile Ala Ala Lys Phe Asp Glu Ala Gln Gln Glu Ala Phe Tyr Trp Ile
1               5                   10                  15

Leu His Leu Glu Asn Leu Thr Glu Glu Gln Arg Asn Ala Phe Ile Gln
            20                  25                  30

Ser Leu Arg Asp Asp Pro Ser Val Ser Glu Glu Val Leu Gly Glu Ala
        35                  40                  45

Leu Lys Leu Asn Val Ser Gln Ala Pro Lys Ile Ala Ala Lys Phe Asp
    50                  55                  60

Glu Ala Gln Tyr Ala Ala Phe Trp Glu Ile Gln His Leu Pro Asn Leu
65                  70                  75                  80

Thr Glu Glu Gln Arg His Ala Phe Ile Trp Ser Leu Leu Asp Asp Pro
                85                  90                  95

Ser Val Ser Leu Glu Val Leu Gly Glu Ala Gln Lys Leu Asn Asp Ser
            100                 105                 110

Gln Ala Pro Lys
        115


<210>  22
<211>  117
<212>  PRT
<213>  Artificial Sequence

<220>
<223>  CFH binding protein 209921

<400>  22

Ile Ala Ala Lys Phe Asp Glu Ala Gln Gln Glu Trp Ala Phe Tyr His
1               5                   10                  15

Ile Leu His Leu Tyr Asn Leu Thr Glu Glu Gln Arg Asn Ala Phe Ile
            20                  25                  30

Gln Ser Leu Arg Asp Asp Pro Ser Val Ser Gln Glu Val Leu Gly Glu
        35                  40                  45

Ala Trp Lys Leu Asn Gln Ser Gln Ala Pro Lys Ile Ala Ala Lys Phe
    50                  55                  60

Asp Glu Ala Gln Tyr Ala Ala Phe Trp Glu Ile Trp His Leu Pro Asn
65                  70                  75                  80

Leu Thr Glu Glu Gln Arg Trp Ala Phe Ile Leu Ser Leu His Asp Asp
                85                  90                  95

Pro Ser Val Ser Leu Glu Val Leu Gly Glu Ala Gln Lys Leu Asn Asp
            100                 105                 110

Ser Gln Ala Pro Lys
        115

<210>  23
<211>  114
<212>  PRT
<213>  Artificial Sequence

<220>
<223>  CFH binding protein 213108 (SEQ13/209621variant del2N)

<400>  23

Ala Lys Phe Asp Glu Ala Gln Thr Ala Ala Phe Trp Glu Ile Glu His
1               5                   10                  15

Leu Pro Asn Leu Thr Glu Glu Gln Arg Trp Ala Phe Ile Trp Ser Leu
            20                  25                  30

Ala Asp Asp Pro Ser Val Ser Leu Glu Val Leu Gly Glu Ala Gln Lys
        35                  40                  45

Leu Asn Asp Ser Gln Ala Pro Lys Ile Ala Ala Lys Phe Asp Glu Ala
    50                  55                  60

Gln Gln Ala Ala Phe Tyr Glu Ile Leu His Leu Pro Asn Leu Thr Glu
65                  70                  75                  80

Trp Gln Arg Asn Tyr Phe Ile Gln Lys Leu Arg Asp Asp Pro Ser Val
                85                  90                  95

Ser Leu Trp Val Leu Gly Gln Ala Gln Ile Leu Asn Asp Ser Gln Ala
            100                 105                 110

Pro Lys

<210>  24
<211>  250
<212>  PRT
<213>  Artificial Sequence

<220>
<223>  CFH binding protein 213109 (209621 delN dimer)

<400>  24

Ala Lys Phe Asp Glu Ala Gln Thr Ala Ala Phe Trp Glu Ile Glu His
1               5                   10                  15

Leu Pro Asn Leu Thr Glu Glu Gln Arg Trp Ala Phe Ile Trp Ser Leu
            20                  25                  30

Ala Asp Asp Pro Ser Val Ser Leu Glu Val Leu Gly Glu Ala Gln Lys
        35                  40                  45

Leu Asn Asp Ser Gln Ala Pro Lys Ile Ala Ala Lys Phe Asp Glu Ala
    50                  55                  60

Gln Gln Ala Ala Phe Tyr Glu Ile Leu His Leu Pro Asn Leu Thr Glu
65                  70                  75                  80

Trp Gln Arg Asn Tyr Phe Ile Gln Lys Leu Arg Asp Asp Pro Ser Val
                85                  90                  95

Ser Leu Trp Val Leu Gly Gln Ala Gln Ile Leu Asn Asp Ser Gln Ala
            100                 105                 110

Pro Lys Gln Ala Pro Lys Val Asp Ala Lys Phe Asp Gln Ala Pro Lys
        115                 120                 125

Val Asp Ala Lys Phe Asp Ile Ala Ala Lys Phe Asp Glu Ala Gln Thr
    130                 135                 140

Ala Ala Phe Trp Glu Ile Glu His Leu Pro Asn Leu Thr Glu Glu Gln
145                 150                 155                 160

Arg Trp Ala Phe Ile Trp Ser Leu Ala Asp Asp Pro Ser Val Ser Leu
                165                 170                 175

Glu Val Leu Gly Glu Ala Gln Lys Leu Asn Asp Ser Gln Ala Pro Lys
            180                 185                 190

Ile Ala Ala Lys Phe Asp Glu Ala Gln Gln Ala Ala Phe Tyr Glu Ile
        195                 200                 205

Leu His Leu Pro Asn Leu Thr Glu Trp Gln Arg Asn Tyr Phe Ile Gln

210                 215                 220

Lys Leu Arg Asp Asp Pro Ser Val Ser Leu Trp Val Leu Gly Gln Ala
225                 230                 235                 240

Gln Ile Leu Asn Asp Ser Gln Ala Pro Lys
                245                 250

<210>  25
<211>  288
<212>  PRT
<213>  Artificial Sequence

<220>
<223>  CFH binding protien 213110 (209621 delN fusion with PAdelFc
       trimer)

<400>  25

Ala Lys Phe Asp Glu Ala Gln Thr Ala Ala Phe Trp Glu Ile Glu His
1               5                   10                  15

Leu Pro Asn Leu Thr Glu Glu Gln Arg Trp Ala Phe Ile Trp Ser Leu
            20                  25                  30

Ala Asp Asp Pro Ser Val Ser Leu Glu Val Leu Gly Glu Ala Gln Lys
        35                  40                  45

Leu Asn Asp Ser Gln Ala Pro Lys Ile Ala Ala Lys Phe Asp Glu Ala
    50                  55                  60

Gln Gln Ala Ala Phe Tyr Glu Ile Leu His Leu Pro Asn Leu Thr Glu
65                  70                  75                  80

Trp Gln Arg Asn Tyr Phe Ile Gln Lys Leu Arg Asp Asp Pro Ser Val
                85                  90                  95

Ser Leu Trp Val Leu Gly Gln Ala Gln Ile Leu Asn Asp Ser Gln Ala
            100                 105                 110

Pro Lys Ile Ala Ala Gln His Asp Lys Ile Gln Gln Ala Ala Asp Lys
        115                 120                 125

Glu Ile Leu His Leu Pro Asn Leu Thr Glu Glu Gln Arg Asn Lys Phe
    130                 135                 140

Arg Gln Ser Leu Arg Asp Asp Pro Ser Val Ser Ala Glu Ile Leu Ala
145                 150                 155                 160

Glu Ala Lys Lys Leu Asn Asp Ala Gln Ala Pro Lys Ile Ala Ala Gln

165                 170                 175

His Asp Lys Ile Gln Gln Ala Ala Asp Lys Glu Ile Leu His Leu Pro
            180                 185                 190

Asn Leu Thr Glu Glu Gln Arg Asn Lys Phe Arg Gln Ser Leu Arg Asp
        195                 200                 205

Asp Pro Ser Val Ser Ala Glu Ile Leu Ala Glu Ala Lys Lys Leu Asn
    210                 215                 220

Asp Ala Gln Ala Pro Lys Ile Ala Ala Gln His Asp Lys Ile Gln Gln
225                 230                 235                 240

Ala Ala Asp Lys Glu Ile Leu His Leu Pro Asn Leu Thr Glu Glu Gln
                245                 250                 255

Arg Asn Lys Phe Arg Gln Ser Leu Arg Asp Asp Pro Ser Val Ser Ala
            260                 265                 270

Glu Ile Leu Ala Glu Ala Lys Lys Leu Asn Asp Ala Gln Ala Pro Lys
        275                 280                 285

<210>  26
<211>  58
<212>  PRT
<213>  Artificial Sequence

<220>
<223>  PAdelFc (non Ig binding protein)

<400>  26

Ile Ala Ala Gln His Asp Lys Ile Gln Gln Ala Ala Asp Lys Glu Ile
1               5                   10                  15

Leu His Leu Pro Asn Leu Thr Glu Glu Gln Arg Asn Lys Phe Arg Gln
            20                  25                  30

Ser Leu Arg Asp Asp Pro Ser Val Ser Ala Glu Ile Leu Ala Glu Ala
        35                  40                  45

Lys Lys Leu Asn Asp Ala Gln Ala Pro Lys
    50                  55

<210>  27
<211>  11
<212>  PRT
<213>  Artificial Sequence

<220>

<223>  coupling and Streptag

<400>  27

Cys Ser Ala Trp Ser His Pro Gln Phe Glu Lys
1               5                   10


<210>  28
<211>  10
<212>  PRT
<213>  Artificial Sequence

<220>
<223>  streptag

<400>  28

Ser Ala Trp Ser His Pro Gln Phe Glu Lys
1               5                   10

## Description

### TECHNICAL FIELD

The present invention relates to novel proteins that specifically bind to complement factor H (CFH). The present invention relates to the field of the purification of CFH. The novel proteins of the present invention are advanced and powerful tools because they allow precise capturing of CFH in affinity chromatography.

### BACKGROUND OF THE INVENTION

Complement factor H (CFH; beta-1H globulin) is a serum glycoprotein naturally occurring in the blood plasma. CFH is the major fluid phase regulator of the alternative pathway of the complement system, preventing the formation of C3 and C5 convertases and facilitating the disassembly of already formed convertases. Importantly, CFH acts as a cofactor for the inactivation of C3b by enzymatic cleavage. If due to a mutation of CFH, C3b is not inactivated, the result might be severe diseases, for example kidney damage. Other complications might be possible. Factor H is known for therapeutic applications. In order to supply Factor H, the factor must be provided in highly purified and active form.

However, current purification process of CFH (or recombinant CFH) involve a complicated prodecure of several purification steps. For example, one known purification of CFH is a three - step chromatography purification (hydrophobic interaction, affinity-chromatography with Heparin, and Ion exchange chromatography). The yield after method of purification is very low (only about 15%) and only a small-scale purification is possible.

There is a strong need in the art to provide methods for a more simple and efficient purification of CFH. The present invention meets this need by providing novel binding proteins for CFH. These novel binding proteins are particularly advantageous because they allow a precise capturing of factor H in affinity chromatography as well as diagnostic applications. This will open successful purification of CFH, for example, for quantities economically viable for medical purposes.

The above overview does not necessarily describe all problems solved by the present invention.

### SUMMARY OF THE INVENTION

The present disclosure provides the following items 1 to 15, without being specifically limited thereto:
1. A binding protein for complement factor H (CFH) comprising an amino acid sequence with at least 80 % sequence identity to any one selected from the group of SEQ ID NOs: 1-25 wherein the binding protein has a binding affinity of less than 1 µM for CFH.
2. A binding protein for CFH according to item 1 wherein the CFH binding protein has a binding affinity of less than 500 nM for CFH.
3. The binding protein for CFH according to items 1-2, wherein 2, 3, 4, 5, or 6 CFH binding proteins are linked to each other.
4. The binding protein for CFH according to item 3, wherein the binding protein is a homo-multimer or a hetero-multimer.
5. The binding protein for CFH according to items 1-4 wherein the binding protein is comprising additionally at least one further molecule, preferably selected from the group of (a) non-Immunoglobulin (Ig)-binding protein, or (b) a diagnostically active moiety, optionally selected from a radionuclide, fluorescent protein, photosensitizer, dye, or enzyme, or any combination of the above.
6. The binding protein for CFH according to any one of items 1-5 for use in in the diagnosis of diseases related to CFH
7. The binding protein for CFH according to any one of items 1-6 for use in technical applications such as affinity purification of CFH or a protein comprising CFH.
8. An affinity separation matrix comprising a binding protein for CFH according to any one of items 1-7.
9. Use of the binding protein for CFH according to any one of items 1-7, or the affinity separation matrix according to item 8 for affinity purification of CFH or a protein comprising CFH.
10. A process of manufacturing CFH comprising at least one chromatographic step employing an affinity chromatography matrix having an affinity for specifically binding CFH wherein the binding protein for CFH according to any one of items 1-7 is coupled to said affinity chromatography matrix.
11. A method of affinity purification of CFH, the method comprising: (a) providing a liquid that contains a CFH; (b) providing an affinity separation matrix comprising at least one binding protein for CFH according to any one of items 1-7 coupled to said affinity separation matrix; (c) contacting said affinity separation matrix with the liquid under conditions that permit binding of the at least one binding protein for CFH according to any one of items 1-7; and (d) eluting said CFH from said affinity purification matrix.
12. Use of the binding protein for CFH according to items 1-7, in methods to determine the presence of CFH.
13. A method of analyzing the presence of CFH in liquid samples, the method comprising the following steps:
   (i) providing a liquid that contains CFH,
   (ii) providing the binding protein for CFH according to items 1-7,
   (iii) contacting the liquid of (i) with the binding protein for CFH according to items 1-7 under conditions that permit binding of the binding protein to the CFH,
   (iv) isolating the complex of CFH according to items 1-7, and
   (v) determining the amount of the binding protein for CFH according to items 1-7 in the liquid of (i).
14. A polynucleotide encoding the binding protein according to any one of items 1-7.

This summary of the invention is not limiting, and other aspects and embodiments of the invention will become evident from the following description, examples and drawings.

### BRIEF DESCRIPTION OF THE FIGURES

**FIGURE 1****:** Amino acid sequences of binding proteins for CFH.
**FIGURE 2****:** Analytical results of CFH binding proteins (binding to CFH and purification)
**FIGURE 3****:** SE-HPLC of CFH that purified via Praesto with immobilized CFH affinity ligand 209621 (dimer; CID213108); lane 7 shows the eluted fraction of CFH (elution at pH 3.5)
**FIGURE 4****:** Binding of purified CFH vs. immobilized CFH affinity ligand. Shown is a SE_HPLC chromatogram of CFH which was eluted from Praesto-213108 with immobilized CFH affinity ligand 209621 (dimer; CID213108). Integrity of CFH was confirmed after purification via Praesto-213108.
**FIGURE 5****:** Caustic stability of affinity ligand 209621 (dimer). Shown is the UV208 nm breakthrough profile. The figure shows no significant reduction in dynamic binding capacity of the resin after 12.5 h 0.1 M NaOH incubation. The NaOH stability of the affinity ligand was 98.9%.

### DETAILED DESCRIPTION OF THE INVENTION

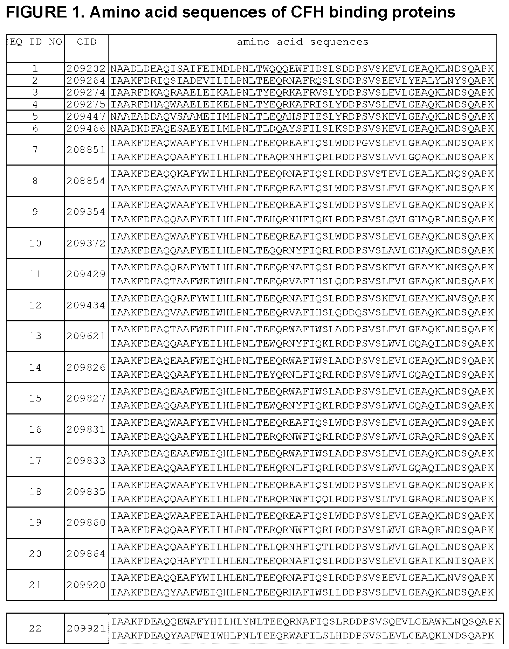

The present invention provides novel proteins having specific binding affinity for CFH including a variant or a domain thereof. The novel proteins of the present invention are particularly advantageous because as affinity ligands for CFH, the allow precise purification of CFH in affinity chromatography. Further, the novel proteins of the present invention can be used in medical applications related to CFH. Any polypeptide selected from the group of SEQ ID NOS: 1-25, or an amino acid sequence with at least 80 % identity to any one of SEQ ID NOs: 1-25, binds to CFH. The amino acid sequences of binding proteins for CFH are shown in **FIGURE 1****.**

Before the present invention is described in more detail below it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is reflected by the appended items. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new specific binding molecules for CFH for use in technical applications, for example for use as affinity ligands in affinity chromatography.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the items, which follow, unless the context requires otherwise, the word "comprise", and variants such as "comprises" and "comprising", will be understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

Several documents (for example: patents, patent applications, scientific publications, manufacturer's specifications, instructions, GenBank Accession Number, etc.) may be cited throughout the present specification. Nothing herein is to be construed as an admission that the invention is not entitled to antedate such disclosure by virtue of prior invention. Some of the documents cited herein may be characterized as being *"incorporated by reference".* In the event of a conflict between the definitions or teachings of such incorporated references and definitions or teachings recited in the present specification, the text of the present specification takes precedence.

All sequences referred to herein are disclosed in the attached sequence listing that, with its whole content and disclosure, forms part of the disclosure content of the present specification.

### General Definitions of Important terms used in the Application

The term "complement factor H" relates to complement factor H (CFH), a domain thereof, and to variants thereof. It refers to an amino acid sequence as shown in UniProtKB Q5TFM2 (SwissProt ID P08603), or variants thereof or fragments or domains thereof. The term "complement factor H" comprises all polypeptides which show an amino acid sequence identity of at least 70 %, 80 %, 85 %, 90 %, 95 %, 96 % or 97 % or more, or 100 % to UniProtKB Q5TFM2. The term also relates to recombinant human complement factor H (or a domain thereof or a variant thereof) produced, for example, in moss (*Physcomitrella patens).* Domains of complement factor H may relate to C3 regulatory domains, for example, N-terminal C3b binding site (Kuhn et al. J. Immunol. 1995; 155:5663-5670).

The term "binding protein for complement factor H" or "CFH binding protein" or "binding protein for CFH" or "affinity ligand for CFH" describes a protein that is capable to bind to complement factor H (CFH) or a variant or a domain thereof, as defined above. As described herein, a binding protein for CFH refers to a protein with detectable interaction with CFH, as determined by suitable methods such as for example SPR analysis or BLI or other appropriate technology known to someone skilled in the art. For example, the term binding protein for CFH includes proteins that bind, for example, to domain 1, 2, 3, 4 of CFH.

The terms "binding affinity" and "binding activity" may be used herein interchangeably and they refer to the ability of a polypeptide of the invention to bind to CFH including a fragment or domain thereof. Binding affinity is typically measured and reported by the equilibrium dissociation constant (K_{D}) which is used to evaluate and rank the strength of bimolecular interactions. The binding affinity and dissociation constants can be measured quantitatively. Methods for determining binding affinities are well known to the skilled person and can be selected, for instance, from the following methods that are well established in the art: surface plasmon resonance (SPR), Bio-layer interferometry (BLI), enzyme-linked immunosorbent assay (ELISA), kinetic exclusion analysis (KinExA assay), flow cytometry, fluorescence spectroscopy techniques, isothermal titration calorimetry (ITC), analytical ultracentrifugation, radioimmunoassay (RIA or IRMA), and enhanced chemiluminescence (ECL). Typically, the dissociation constant K_{D} is determined at temperatures in the range of 20°C and 30°C. If not specifically indicated otherwise, K_{D} values recited herein are determined at 25°C by SPR. The most widely used SPR-based system is the BIAcore, produced by BIAcore AB. In various embodiments of the present invention, the binding affinity for CFH or a domain thereof may be determined by the BIAcore SPR system. The term "fusion protein" relates to a protein comprising at least a first protein joined genetically to at least a second protein. A fusion protein is created through joining of two or more genes that originally coded for separate proteins. Thus, a fusion protein may comprise a multimer of identical or different proteins which are expressed as a single, linear polypeptide.

As used herein, the term "linker" refers in its broadest meaning to a molecule that covalently joins at least two other molecules.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the polypeptide of SEQ ID NO: 13. Methods for sequence alignment are well known in the art. For example, for determining the extent of an amino acid sequence identity of an arbitrary polypeptide relative to the amino acid sequence of, for example, SEQ ID NO: 13, the SIM Local similarity program is preferably employed (Xiaoquin Huang and Webb Miller (1991), Advances in Applied Mathematics, vol. 12: 337-357), that is freely available. For multiple alignment analysis, ClustalW is preferably used (Thompson et al. (1994) Nucleic Acids Res., 22(22): 4673-4680).

The terms "protein" and "polypeptide" refer to any chain of two or more amino acids linked by peptide bonds and does not refer to a specific length of the product. Thus, "peptides", "protein", "amino acid chain", or any other term used to refer to a chain of two or more amino acids, are included within the definition of "polypeptide", and the term "polypeptide" may be used instead of, or interchangeably with, any of these terms. The term "polypeptide" is also intended to refer to the products of post-translational modifications of the polypeptide like, e.g., glycosylation, which are well known in the art.

The term "alkaline stable" or "alkaline stability" or "caustic stable" or "caustic stability" refers to the ability of the binding protein for CFH or a domain thereof to withstand alkaline conditions without significantly losing the ability to bind CFH or a domain thereof. The skilled person in this field can easily test alkaline stability by incubating an CFH or a domain thereof of Binding protein for CFH or a domain thereof with sodium hydroxide solutions, e.g., as described in the Examples, and subsequent testing of the binding activity to CFH or a domain thereof by routine experiments known to someone skilled in the art, for example, by chromatographic approaches.

The term "chromatography" refers to separation technologies which employ a mobile phase and a stationary phase to separate one type of molecules (e.g., CFH or variant or a domain thereof) from other molecules (e.g., contaminants) in the sample. The liquid mobile phase contains a mixture of molecules and transports these across or through a stationary phase (such as a solid matrix). Due to the differential interaction of the different molecules in the mobile phase with the stationary phase, molecules in the mobile phase can be separated.

The term "affinity chromatography" refers to a specific mode of chromatography in which a ligand (i.e. a binding protein for CFH or a domain thereof coupled to a stationary phase interacts with a molecule (i.e. CFH or a domain thereof) in the mobile phase (the sample) i.e. the ligand has a specific binding affinity for the molecule to be purified. As understood in the context of the invention, affinity chromatography involves the addition of a sample containing a CFH or a domain thereof to a stationary phase which comprises a chromatography ligand, such as a binding protein for CFH or a domain thereof. The terms "solid support" or "solid matrix" are used interchangeably for the stationary phase.

The terms "affinity matrix" or "affinity purification matrix" or "affinity chromatography matrix", as used interchangeably herein, refer to a matrix, e.g., a chromatographic matrix, onto which an affinity ligand e.g., a binding protein for CFH is attached. The attached affinity ligand (e.g., binding protein for CFH) is capable of specific binding to a molecule of interest (e.g., CFH or a domain thereof or variants thereof) which is to be purified or removed from a mixture.

The term "affinity purification" as used herein refers to a method of purifying a CFH or a domain thereof from a liquid by binding CFH or a domain thereof to binding protein for CFH or a domain thereof that is immobilized to a matrix. Thereby, other components of the mixture except CFH or a domain thereof are removed. In a further step, the bound protein can be eluted in highly purified form.

### Detailed description of the embodiments of the invention

The present invention will now be further described. In the following passages different aspects of the invention are defined in more detail. Each aspect defined below may be combined with any other aspect or aspects unless clearly indicated to the contrary. In particular, any feature indicated as being preferred or advantageous may be combined with any other feature or features indicated as being preferred or advantageous.

The novel binding protein for CFH exhibit a specific binding affinity for the CFH. The binding protein for CFH comprises the amino acid sequence selected from the group of SEQ ID NOs: 1-25 or an amino acid with at least 80 % sequence identity to any one of SEQ ID NOs: 1-25. In some embodiments, a binding protein for CFH is comprising at least one amino acid sequence as shown in **FIGURE 1****.**

In some embodiments, the binding protein for CFH has at least 80 %, 81 %, 82 %, 83 %, 84 %, 85 %, 86 %, 87 %, 88 %, 89 %, 89 %, 91 %, 92 %, 93 %, 94 %, 95%, 96 %, 97 %, 98 %, 99 %, or 100 % sequence identity to any one of the amino sequences of SEQ ID NOs: 1-25.

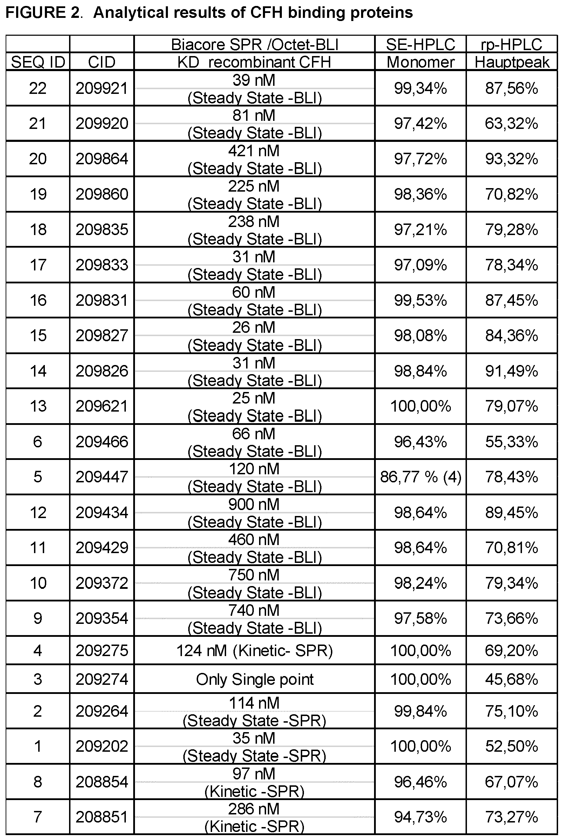

One advantage of the disclosed binding protein for CFH is the important functional characteristic that it binds specifically to CFH. Needless to point out, that this is of particular advantage in the purification of proteins CFH. The binding protein for CFH is functionally characterized by a binding affinity of less than 1 µM for CFH, as shown in the **FIGURE 2** and in **Example 3.**

A common structural feature of the binding proteins is that they are based on artificial mosaic proteins that are stable under conditions as usually applied in affinity chromatography, for example, under alkaline conditions. For example, the general scaffold of the CFH binding protein is a triple helical structure. If the CFH binding protein is a dimer, the general scaffold has a triple helical structure in each monomer. The artificial mosaic proteins are composed of fragments of several wild-type Protein A (SpA) domains and have further specific mutations that generate the unique and surprising CFH binding functionality.

**Multimers.** In one embodiment of the invention, the binding protein for CFH comprises 1, 2, 3, 4, 5, or 6 binding protein(s) linked to each other. For example, SEQ ID NOs: 1-6, 25 are monomers, SEQ ID NOs: 7-24 are dimers. Multimers of the binding protein are generated artificially, generally by recombinant DNA technology well-known to a skilled person. In some embodiments, the multimer is a homo-multimer, e.g. the amino acid sequences of binding protein for CFH are identical. For example, SEQ ID NO: 23 is a homo-dimer of SEQ ID NO: 13.

In other embodiments, the multimer is a hetero-multimer, e.g. the amino acid sequences of the binding protein for CFH are different. For example, SEQ ID NOs: 7-22 are hetero-dimer composed of two different monomers. In some embodiments, these monomers are directly joined.

**Additional moieties.** In some embodiments, the binding protein for CFH as described above further comprises at least one further polypeptide distinct from the polypeptide as disclosed. In various embodiments, the further polypeptide distinct from the binding protein for CFH as disclosed herein might be a non-Ig-binding protein, for example but not limited to, a protein that does not bind to the Fc part of immunoglobulin. In some embodiments, a non-Ig binding protein has at least 89.5 % identity to SEQ ID NO: 26. Accordingly, some embodiments encompass fusion proteins comprising a binding protein for CFH as disclosed herein and one or two or more non-Ig-binding polypeptide(s). In some embodiments, fusion proteins comprising at least one binding protein for CFH and at least one non-Ig binding protein are provided in SEQ ID NO: 25.

In one embodiment, CID209621 fused to at least one a non-Ig binding protein as shown in SEQ ID NO: 25 (CID21310).

In some embodiments, the binding protein for CFH as described above further comprises at least one further polypeptide distinct from the polypeptide as disclosed. In various embodiments, the further polypeptide distinct from the binding protein for CFH as disclosed herein might be at least one diagnostically active moiety, optionally selected from a radionuclide, fluorescent protein, photosensitizer, dye, or enzyme, or any combination of the above. In some embodiments, a binding protein for CFH that comprises additionally at least one diagnostic moiety can be employed, for example, as imaging agent. Suitable radionuclides for applications in imaging *in vivo or in vitro* or for radiotherapy include for example but are not limited to the group of gamma-emitting isotopes, the group of positron emitters, the group of beta-emitters, and the group of alpha-emitters. In some embodiments, suitable conjugation partners include chelators such as 1,4,7,10-tetraazacyclododecane-1,4,7,10-tetraacetic acid (DOTA) or diethylene triamine pentaacetic acid (DTPA) or their activated derivatives, nanoparticles and liposomes. In various embodiments, DOTA may be suitable as complexing agent for radioisotopes and other agents for imaging.

In some embodiments, the binding protein for CFH as described above further comprises at least one further polypeptide distinct from the polypeptide as disclosed. In various embodiments, the further polypeptide distinct from the binding protein for CFH as disclosed herein might be at least one therapeutically active moiety, optionally selected from a monoclonal antibody or a fragment thereof, a radionuclide, a cytotoxic compound, a cytokine, a chemokine, an enzyme, or derivatives thereof, or any combination of the above. In some embodiments, the binding protein for CFH as described above additionally comprises a therapeutically active component and may be used in targeted delivery of any of the above listed components to the CFH.

In some embodiments, the binding protein for CFH as described above further comprises at least one further polypeptide distinct from the polypeptide as disclosed. In various embodiments, the further polypeptide distinct from the binding protein for CFH as disclosed herein might be at least one moiety modulating pharmacokinetics optionally selected from a polyethylene glycol, a human serum albumin, an albumin-binding protein or peptide, an immunoglobulin binding protein or peptide, or an immunoglobulin or immunoglobulin fragment, a polysaccharide, or an unstructured amino acid sequence comprising amino acids alanine, glycine, serine, proline.

The moieties may be linked to each other directly head-to-tail or may be linked by a linker, wherein the linker preferably is a peptide linker. In various embodiments, a peptide linker may be considered as an amino acid sequence which sterically separates the two portions of the fusion protein. Typically, such linker consists of between 1 and 30 amino acids. In some embodiments, the linker may consist of 20 amino acids selected from any amino acid. In some embodiments, the linker may consist of 20 amino acids selected from the group of Q, A, P, K, V, D, F, S (see, for example, in the fusion protein of SEQ ID NO: 24)

**Molecules for purification or detection.** In some embodiments, the binding protein for CFH may also comprise additional amino acid residues at the N- and/or C-terminal end, such as for example an additional sequence at the N- and/or C-terminal end. Additional sequences may include for example sequences introduced e.g. for purification or detection. Typical examples for such sequences include, without being limiting, Strep-tags, oligohistidine-tags, glutathione S-transferase, maltose-binding protein, inteins, intein fragments, or the albumin-binding domain of protein G, or others. In one embodiment, additional amino acid sequences include one or more peptide sequences that confer an affinity to certain chromatography column materials. The binding protein for CFH may include specific attachment sites for the attachment to solid supports, preferably at the C-terminal end, such as cysteine or lysine.

**Method of identification the binding protein for CFH.** The present invention further provides a method for the identification of a binding polypeptide CFH as disclosed herein with binding affinity for CFH, the method comprising the following steps: (i) providing a population (library) of proteins; (ii) contacting the population of proteins of (i) with a CFH; (iii) identifying a complex comprising an binding protein for CFH and CFH; and (iv) obtaining an binding protein for CFH.

The method for the identitification of a binding protein for CFH may comprise, a further step of determining the binding affinity to CFH. The binding affinity may be determined as described elsewhere herein.

**Use of the novel binding protein for CFH or a domain thereof in technical applications.** Also provided herein is the use of any novel binding protein for CFH as disclosed herein, including multimers, including fusion proteins, in technical applications, preferably for use in affinity purification.

As described herein, affinity chromatography (also called affinity purification) makes use of specific binding interactions between molecules. Methods for immobilization of protein and methods for affinity chromatography are well-known in the field of protein purification and can be easily performed by a skilled person in this field using standard techniques and equipment.

In various embodiments, the method of affinity purification may further comprise one or more washing steps carried out under conditions sufficient to remove from the affinity purification matrix some or all molecules that are non-specifically bound thereto. Affinity purification matrices suitable for the disclosed uses and methods are known to a person skilled in the art. **Conjugation to a solid support.** In various aspects and/or embodiments of the present invention, the novel proteins disclosed herein including novel proteins generated or obtained by any of the methods as described above are conjugated to a solid support. In some embodiments of the invention, the polypeptide comprises an attachment site for site-specific covalent coupling of the polypeptide to a solid support. Specific attachment sites comprise without being limited thereto, natural amino acids, such as cysteine or lysine, which enable specific chemical reactions with a reactive group of the solid phase, or a linker between the solid phase and the protein. **Affinity purification matrix.** In another embodiment, an affinity purification matrix is provided comprising a binding protein for CFH, including a polypeptide identified by any of the methods as described above.

In preferred embodiments, the affinity purification matrix is a solid support. The affinity purification matrix comprises at least one binding protein for CFH as described herein. Accordingly, a novel binding protein for CFH disclosed herein is encompassed for use in the purification of CFH by an affinity matrix.

Solid support matrices for affinity chromatography are known in the art and include, *e.g.,* without being limited thereto, agarose and stabilized derivatives of agarose, cellulose or derivatives of cellulose, controlled pore glass, monolith, silica, zirconium oxide, titanium oxide, or synthetic polymers, and hydrogels of various compositions and combinations of the above.

The formats for solid support matrices can be of any suitable well-known kind. Such solid support matrix for coupling a novel protein or polypeptide of the present invention might comprise, *e.g.,* one of the following, without being limited thereto: columns, capillaries, particles, membranes, filters, monoliths, fibers, pads, gels, slides, plates, cassettes, or any other format commonly used in chromatography and known to someone skilled in the art.

In one embodiment, the matrix is comprised of substantially spherical beads, for example Sepharose or Agarose beads. Matrices in particle form can be used as a packed bed or in a suspended form including expanded beds. In other embodiments of the invention, the solid support matrix is a membrane, for example a hydrogel membrane. In some embodiments, the affinity purification may involve a membrane as a matrix to which a binding protein for CFH of the present invention is covalently bound. The solid support can also be in the form of a membrane in a cartridge.

In some embodiments, the affinity purification involves a chromatography column containing a solid support matrix to which a novel protein of the present invention is covalently bound. A novel protein or polypeptide of the present invention may be attached to a suitable solid support matrix via conventional coupling techniques. Methods for immobilization of protein ligands to solid supports are well-known in the field of protein engineering and purification and can easily be performed by a skilled person in this field using standard techniques and equipment.

Further embodiments relate to a process of manufacturing CFH comprising at least one chromatographic step employing an affinity chromatography matrix having an affinity for specifically binding CFH wherein the binding protein for CFH as described above is coupled to said affinity chromatography matrix.

Further, in some embodiments, the binding protein for CFH as described herein or the fusion protein as described herein are used in methods to determine the presence of a CFH. Some embodiments relate to a method of analyzing the presence of CFH in liquid samples, the method comprising the following steps: (a) providing a liquid that contains a CFH, (b) providing the binding protein for CFH, (c) contacting the liquid that contains CFH with the binding protein for CFH as described herein under conditions that permit binding of the at least one binding protein for CFH to CFH, (d) isolating (eluting) the complex of a CFH and the binding protein for CFH, and optionally, (e) determining the amount of the binding protein for CFH which indicates the amount of CFH in the liquid of (a).

Further embodiments relate to a method of quantification of a CFH, the method comprising: (a) providing a liquid that contains CFH; (b) providing a matrix to which the binding protein for CFH as described herein has been covalently coupled; (c) contacting said affinity purification matrix with the liquid under conditions that permit binding of the at least one binding protein for CFH to CFH; (d) eluting said CFH; and optionally, (e) quantitating the amount of eluted CFH. Methods to determine the presence of CFH in liquid samples might be quantitative or qualitative. Such methods are well known to the skilled person and can be selected, for instance but limited to, from the following methods that are well established in the art: enzyme-linked immunosorbent assay (ELISA), enzymatic reactions, surface plasmon resonance (SPR) or chromatography. **Medical applications.** In some embodiments, the binding protein for CFH as described above is used in diagnosis or treatment of CFH related diseases. In one embodiment, the binding protein for CFH is used in medicine to diagnose of CFH or treat diseases associated with CFH.

One embodiment is a method of diagnosing (including monitoring), the method of diagnosis (monitoring) comprising administering to the subject the binding protein for CFH as described, optionally conjugated to radioactive molecules. In various embodiments, the binding protein for CFH as disclosed herein may be used for diagnosis of CFH, optionally wherein the binding protein for CFH o is conjugated to a radioactive molecule. In some embodiments, imaging methods using the binding protein for CFH with labels such as radioactive or fluorescent can be employed. One embodiment is a method of treating a subject having diseases related to or caused by CFH, the method of treatment comprising administering to the subject the CFH binding protein as described herein. In various embodiments, the binding protein for CFH as disclosed herein may be used for treatment of diseases related to or caused by CFH.

**Compositions.** Various embodiments relate to a composition comprising the binding protein for CFH as disclosed herein. A composition comprising the binding protein for CFH as defined above for use in medicine, preferably for use in the diagnosis (detection / monitoring) of CFH or treatment of diseases related to or caused by CFH as described above. Compositions comprising the binding protein for CFH as described above may be used for clinical applications for both diagnostic and therapeutic purposes. In particular, compositions comprising the binding protein for CFH as described herein may be used for clinical applications for imaging, monitoring, and eliminating or inactivating CFH.

Various embodiments relate to a diagnostic composition for the diagnosis of CFH comprising the binding protein for CFH as defined herein and a diagnostically acceptable carrier and/or diluent. These include for example but are not limited to stabilizing agents, surface-active agents, salts, buffers, coloring agents etc. The compositions can be in the form of a liquid preparation, a lyophilisate, granules, in the form of an emulsion or a liposomal preparation.

The diagnostic composition comprising the binding protein for CFH as described herein can be used for diagnosis of CFH, as described above.

Various embodiments relate to a pharmaceutical (e.g. therapeutical) composition for the treatment of diseases comprising the binding protein for CFH as disclosed herein, and a pharmaceutically (e.g. therapeutically) acceptable carrier and/or diluent. The pharmaceutical (e.g. therapeutical) composition optionally may contain further auxiliary agents and excipients known *per se.* These include for example but are not limited to stabilizing agents, surface-active agents, salts, buffers, coloring agents etc.

The pharmaceutical composition comprising the binding protein for CFH as defined herein can be used for treatment of diseases, as described above.

The compositions contain an effective dose of the binding protein for CFH as defined herein. The amount of protein to be administered depends on the organism, the type of disease, the age and weight of the patient and further factors known *per se.* Depending on the galenic preparation these compositions can be administered parentally by injection or infusion, systemically, intraperitoneally, intramuscularly, subcutaneously, transdermally, or by other conventionally employed methods of application.

The composition can be in the form of a liquid preparation, a lyophilisate, a cream, a lotion for topical application, an aerosol, in the form of powders, granules, in the form of an emulsion or a liposomal preparation. The type of preparation depends on the type of disease, the route of administration, the severity of the disease, the patient and other factors known to those skilled in the art of medicine.

The various components of the composition may be packaged as a kit with instructions for use.

**Polynucleotides, vectors, host cells.** One embodiment covers an isolated polynucleotide or nucleic acid molecule encoding a binding protein for CFH as disclosed herein. A further embodiment also encompasses proteins encoded by the polynucleotides as disclosed herein. Further provided is a vector, in particular an expression vector, comprising the isolated polynucleotide or nucleic acid molecule of the invention, as well as a host cell comprising the isolated polynucleotide or the expression vector. For example, one or more polynucleotides, which encode a polypeptide as disclosed herein may be expressed in a suitable host and the produced protein can be isolated. A vector means any molecule or entity (*e.g.,* nucleic acid, plasmid, bacteriophage or virus) that can be used for transfer of protein-encoding information into a host cell. Suitable vectors that may be applied in the present invention are known in the art. Furthermore, an isolated cell comprising a polynucleotide or nucleic acid, or a vector as disclosed herein is provided. Suitable host cells include prokaryotes or eukaryotes, for example a bacterial host cell, a yeast host cell or a non-human host cell carrying a vector. Suitable bacterial expression host cells or systems are known in the art. Various mammalian or insect cell culture systems as known in the art can also be employed to express recombinant proteins.

**Method of producing a protein of the invention.** In a further embodiment, a method for the production of the binding protein for CFH as described is provided, the method comprising the step(s): (a) culturing a (suitable) host cell under conditions suitable for the expression of the binding protein for CFH so as to obtain said binding protein for CFH or a domain thereof; and (b) optionally isolating said binding protein for CFH. Suitable conditions for culturing a prokaryotic or eukaryotic host are well known to a person skilled in the art.

The binding protein for CFH may be prepared by any conventional and well-known techniques such as plain organic synthetic strategies, solid phase-assisted synthesis techniques, or by commercially available automated synthesizers. They may also be prepared by conventional recombinant techniques, alone or in combination with conventional synthetic techniques.

In one embodiment, a method for the preparation of the binding protein for CFH is provided, as detailed above, said method comprising the steps: (a) providing a nucleic acid molecule encoding the binding polypeptide; (b) introducing said nucleic acid molecule into an expression vector; (c) introducing said expression vector into a host cell; (d) culturing the host cell in a culture medium; (e) subjecting the host cell to culturing conditions suitable for expression thereby producing a binding polypeptide; optionally (f) isolating the protein or polypeptide produced in step (e); and (g) optionally conjugating the protein or polypeptide to a solid matrix as described above. In various embodiments of the present invention the production of the binding protein for CFH is performed by cell-free *in vitro* transcription and translation.

The following Examples are provided for further illustration of the invention. The invention, however, is not limited thereto, and the following Examples merely show the practicability of the invention on the basis of the above description.

### EXAMPLES

The following Examples are provided for further illustration of the invention. The invention, however, is not limited thereto, and the following Examples merely show the practicability of the invention on the basis of the above description. For a complete disclosure of the invention reference is made also to the literature cited in the application which is incorporated completely into the application by reference.

### EXAMPLE 1. Selection and Screening of Binding protein for CFH

*Libraries.* Proprietary cDNA libraries based on stable Protein A like variants (artifical mosaic proteins composed of fragments of Protein A domains and additional mutations) were synthesized in house by randomized oligonucleotides generated by synthetic trinucleotide phosphoramidites (ELLA Biotech) to achieve a well-balanced amino acid distribution with simultaneously exclusion of cysteine and other amino acid residues at randomized positions. The corresponding cDNA library was amplified by PCR and ligated into a pCD33-OmpA phagemid. Aliquots of the ligation mixture were used for electroporation of *E. coli* SS320 (Lucigen) to produce and purify the phage library to store them as cryo-stocks. Unless otherwise indicated, established recombinant genetic methods were used.

*Selection by phage display:* naive libraries were enriched against recombinant human CFH (isolated from moss; also referred to as "moss-FH" herein) as ON-target using phage display as selection system. In each round a pre-selection step was performed using empty Sigmablocker-blocked magnetic beads. The AIT-method was applied, which means that the ON-target protein was immobilized either to magnetic Epoxy M-270 Dynabeads or magnetic Pierce NHS-beads before each round started. *E. coli* ER2738 (Lucigene) were used for infection with cryo phage libraries and for reamplification of phage pools after each round. Amplification and purification of the phages were carried out using standard methods known to a skilled person. All four selection rounds were performed with the automated KingFisher-System (Thermo Fisher) to isolate and capture the desired phage-target complexes. Bound phages were eluted by trypsin and reamplified. The success of the selection was analyzed by phage-pool-ELISA in medium binding microtiter plate (Greiner Bio-One) coated with moss-FH (125 ng/well), BSA (250 ng/well) or Sigmablocker. Bound phages were detected using α-M13 HRP conjugated antibody (GE Healthcare).

*Cloning of target binding phage pools into an expression vector* Selection pools showing specific binding to recombinant CFH in phage pool ELISA were amplified by PCR according to methods known in the art, cut with appropriate restriction nucleases and ligated into a derivative of the expression vector pET-28a (Merck, Germany) comprising a C-terminal cysteine followed by a streptavidin-tag.

*Results:* Various phage display selection pools resulted in specific signals for the respective ON-target CFH. Controls with BSA or Sigmablocker showed for almost all pools no binding. Selected pools were sequenced, subcloned and proceed to high throughput screening. Enriched variants were selected as direct transfer for lab-scale production and purification.

*Primary screening:* Selection pools were proceeded to high through.put primary screening vs. CFH. Therefore CFH [c= 2.5 µg/ml] was immobilized on a 384-well high binding plate and bound variants were detected by using Strep-Tactin^{®} labeled with horseradish peroxidase (absorption at 450/600 nm).

*Secondary screening:* 9063 variants were selected for confirmation screening vs. ON-target: CFH [c= 2.5 µg/ml] and OFF-target: BSA [c= 2.5 µg/ml]. Hit criteria: signal of sample 10-fold larger than signal of OFF-target.

*Tertiary screening:* 3009 variants were defined as hit for screening of low pH stability. Therfore cells were lysed at pH 3.0 with citrate buffer and neutralized with TRIS buffer. Soluble fractions were used for screening vs. ON-target: CFH [c= 2.5 µg/ml] and OFF-target: BSA [c= 2.5 µg/ml]. Hit criteria: signal of variants > 0.7 and 8-fold larger than signal of OFF-target.

*µ-scale purification and BLI analysis:* 456 variants were defined as hit for sequencing, µ-scale purification (PhyNexus, see Example 2) and BLI analysis vs CFH using an Octet 8 channel system. For BLI measurements variants were immobilized via anti-StrepTag antibody on the sensor surface (ForteBio). Upon binding, variants were accumulated on the surface increasing the refractive index. This change in the refractive index was measured in real time and plotted as nm shift versus time. Affinity of captured variants vs CFH [c= 250 nM] was determined and also plotted as nm shift versus time. 27 hit variants were selected for lab scale production.

### EXAMPLE 2. Expression and purification of CFH binding proteins

Variants were expressed in Escherichia coli BL21(DE3) using a pNP-013 vector system under regulation of a T7 promoter. Proteins were produced in soluble form after induction by lactose included in the medium (autoinduction medium). BL21 (DE3) competent cells were transformed with the expression plasmid, spread onto selective agar plates (kanamycin) and incubated overnight at 37°C. Precultures were inoculated from single colony in 3 ml 2xYT medium supplemented with 50 µg/ml kanamycin and cultured for 6 hours at 37 °C at 200 rpm in a conventional orbital shaker in culture tubes. Main cultures were inoculated with 0.3/OD of precultures in 350 ml ZYM-5052 (0.5 % glycerol, 0.2 % lactose, 0.05 % glucose, 0.5 % yeast extract, 1.0 % casamino acids, 25 mM Na₂HPO₄, 25 mM KH₂PO₄, 5 mM Na₂SO₄, 2 mM MgSO₄ and trace elements) in 2.5 L UltraYield flasks. Cultures were transferred to an orbital shaker and incubated at 30 °C and 200 rpm. Recombinant protein expression was induced by metabolizing glucose and subsequently allowing lactose to enter the cells. Cells were grown overnight for approx. 17 hours to reach a final OD600 of about 17-19. Before the harvest, the OD600 was measured, samples adjusted to 0.6/OD600 were withdrawn, pelleted and frozen at -20 °C. To collect biomass cells were centrifuged at 12000 x g for 20 min at 22 °C. Pellets were weighted (wet weight) and stored at -20 °C before processing.

Tagged proteins were purified by affinity and size exclusion chromatography. The initial capturing step was performed using StrepTactin Affinity resin (StrepTactin Superflow 5 ml, IBA, binding buffer: 100 mM TRIS, 150 mM NaCl, 1 mM EDTA, 1 mM DTT, pH 8.0; elution buffer: 100 mM TRIS, 150 mM NaCl, 1 mM EDTA, 1 mM DTT, 2.5 mM D-Desthiobiotin, pH 8.0) followed by a size exclusion chromatography (HiLoad Superdex 75 16/60 120 ml, GE Healthcare) in 20 mM Citric acid, 150 mM NaCl, 1 mM EDTA, 1 mM DTT pH 6.0 using an ÄKTA xpress system. Finally 5 mM TCEP was added to the protein batch. Untagged Proteins were purified by hlgG-Sepharose according to manufactures instructions. Polishing was performed by size exclusion chromatography using a Superdex 75 or 200 26/600 column (GE Healthcare; buffer: 20 mM citric acid, 150 mM NaCl, 1 mM EDTA pH 6) using an ÄKTA avant system (GE Healthcare). Following SDS-PAGE analysis positive fractions were pooled and the protein concentrations were determined by absorbance measurement at 280 nm using the molar absorbent coefficient. Further analysis included RP-HPLC and SE-HPLC. Reversed phase chromatography (RP-HPLC) has been performed using a Dionex HPLC system and a PLRP-S (5 µm, 300 Å) column (Agilent). Analytic size exclusion chromatography (SE-HPLC) has been performed using a Dionex HPLC system and a Superdex75 increase 5/150 GL (GE Healthcare). Results are shown in **FIGURE 2****.**

### EXAMPLE 3. Binding analysis by SPR

Purified proteins were immobilized via C-terminal Cysteine on a CM-5 sensor chip (GE Healthcare) using NHS/EDC after PDEA activation resulting in 110-140 RU with a Biacore 3000 system (GE Healthcare). The chip was equilibrated with SPR running buffer (PBS 0.05 % Tween pH 7.3). Otherwise variants were immobilized via Streptag using rProtein A surface and an anti strep-tag antibody (Anti-NWSHPQFEK, GenScript) for variant capturing.

Soluble recombinant CFH applied to the chip in serial dilutions (different concentrations) with a flow rate of 30 µl/min. The association was performed for 120 seconds and the dissociation for 120 seconds. After each run, the chip surface was regenerated with 30 µl regeneration buffer (10 mM glycine pH 2.0) and equilibrated with running buffer.

CFH accumulated on the surface increasing the refractive index. This change in the refractive index was measured in real time and plotted as response or resonance units versus time.

Binding studies were carried out by the use of the BIAcore 3000 (GE Healthcare); data evaluation was operated via the BIAevaluation 3.0 software, provided by the manufacturer, by the use of the Langmuir 1:1 model (RI=0). Evaluated dissociation constants (K_{D}) were standardized against the immobilized protein and indicated. Shown is the change in refractive index measured in real time and plotted as response or resonance unit [RU] versus time [sec]. Results are shown in **FIGURE 2****.**

### EXAMPLE 4 Kinetic Binding Analysis by Biolayer Interferometry (BLI)

The Octet^{™} system (ForteBio, Pall LLC) was used to determine the binding properties of the affinity ligands for CFH. Protein A sensors (ForteBio) were used for initial loading of anti-strep-tag antibody [c= 150 nM] in 1× kinetics buffer (PBS supplemented with 0.002% Tween-20 and 1 mg/mL of BSA). Upon binding, variants [c= 1µM] were accumulated on the surface increasing the refractive index. The change in the refractive index was measured in real time and plotted as nm shift versus time. Binding to CFH was performed across three-fold serial dilutions of recombinant CFH [cₛₜₐᵣₜ= 1µM]. The baseline and dissociation steps were carried out in the 1× kinetics buffer as per the instrument manufacturer's recommendations. Affinity of captured variants [c= 1 µM] vs CFH was determined and also plotted as nm shift versus time. For all of the kinetics experiments, a global data fitting to a 1:1 binding model was used to estimate values for the kₒₙ (association rate constant), k_{off} (dissociation rate constant), and K_{D} (equilibrium dissociation constant).

### EXAMPLE 5. Affinity purification of CFH

**Coupling parameter.** Proteins were purified to homogeneity and coupled to resin for AIC experiments. Purified binding protein (e.g. 213109, 213109, 213110) for CFH was immobilized at 25 mg or 30 mg per mL activated Praesto^{™} Epoxy 85 (Purolite) according to the manufacturer's instructions, coupling conditions: 35°C for 3 h, pH 9.5, 110 mg Na₂SO₄ per mL Resin. Variants were successfully coupled to epoxy-activated Praesto 85 resin.

**AIC experiments. Capturing and Elution.** For initial column performance tests, resins were packed into superformance 50-5 column housing. Phosphate buffered saline pH 7.3 (PBS) was used to equilibrate the column, followed by applying of 10 mL of recombinant CFH expression supernatant (titer: 0.47 mg/mL) at 6 min residence time. Resin was then washed with PBS for 15 column volumes. Elution was performed at pH 3.5 using 100 mM Citric acid buffer followed by stripping of residual bound CFH at pH 2.0. Elution at pH 3.5 and pH 2.0 was calculated by UV 280 nm absorption. Percentage elution at pH 3.5 is listed in **TABLE 1.**

**TABLE 1. Percentage elution of CFH at pH 3.5**

| **SEQ ID NO:** | **CID** | **Step Yield of CFH at pH 3.5** |
|---|---|---|
| 5 | 209447 | 91 % |
| 10 | 209372 | 58% |
| 11 | 209429 | 96 % |
| 12 | 209434 | 95 % |
| 13 | 209621 | 54 % |
| 19 | 209860 | 77 % |
| 20 | 209864 | 96 % |
| 21 | 209920 | 89 % |
| 22 | 209921 | 91 % |

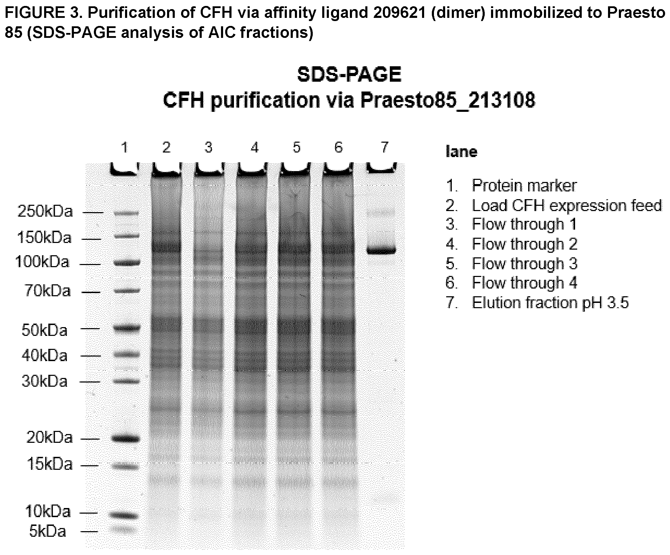

**SBC Determination.** For SBC determination increased volumes (50 ml, titer: 0.75 mg/mL) of recombinant CFH expression supernatant were applied. Dimers of 209621 (SEQ ID NO: 23, 24; CID213108, CID213109) and a fusion protein comprising 209621 (SEQ ID NO: 25, CID213110) showed high step yield at pH 3.5 elution and high purity of CFH in the eluted fractions (SDS-PAGE analysis of AIC fractions see for example **FIGURE 3****).**

The addition of 10% (v/v) hexylene glycol to the elution buffer resulted in increased elution yield at pH 3.5. The final SBC value was determined by SDS-PAGE (software: Totallab). Recombinant CFH was used as standard protein. SDS-PAGE quantification of eluted CFH (eluted from the matrix Praesto_209621) resulted in an SBC value of 27.2 mg/mL. The correlation factor UV280 absorption vs SDS-PAGE was 0.486.

**AIC experiments. DBC and SBC with purified CFH.** DBC Determination was performed with purified CFH and Praesto 85 epoxy activated with immobilized affinity ligand 209621 (referred to as Praesto_209621). Purified CFH was diluted in PBS at 1 mg/ml and adjusted to pH 7.3 and applied onto column at 6 min residence time. Target breakthrough was measured via UV 280 nm absorption until 10% breakthrough for DBC 10% calculation. Target loading was continued until 95% breakthrough followed by column washing with PBS and elution at pH 3.5 in 100 mM Citric acid. Eluted CFH was quantified by UV 280 nm. Capacity values were calculated by mass equation and final corrected by UV 280 nm/SDS-PAGE correlation factor (Results in **TABLE 2)**

**TABLE 2. Capacity values for the dimer of 209621 (CID 213108).**

| **Feed** | **binding capacity** | **determined by UV 280 nm abs (mg/ml)** | **calculated by UV280 nm/SDS PAGE factor (mg/ml)** |
|---|---|---|---|
| purified CFH | SBC | 18.1 | >35.6 |
| purified CFH | DBC @6min residence time | 18,6 | 36.6 |

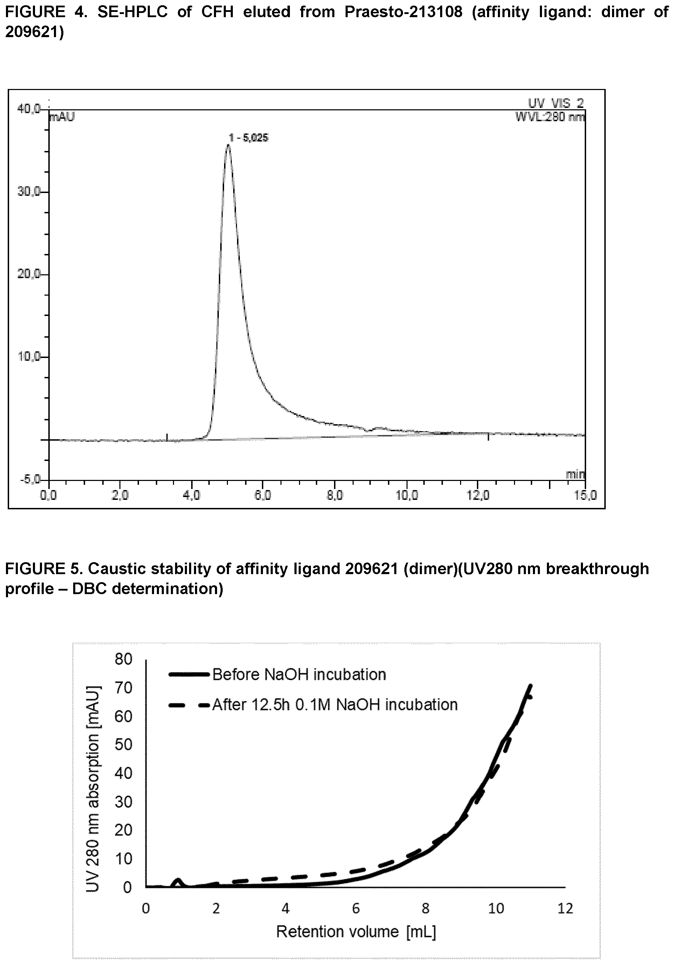

**AIC measurements. Caustic stability.** The dimer of 209621 (CID213108) was coupled to Praesto^{™} Epoxy 85 as described above and treated with 0.1 M NaOH for 12 h at RT (equals 50 cycles). The remaining dynamic binding capacity was determined with recycled target CFH from initial DBC/SBC measurements. **FIGURE 5** confirms that the resin showed no significant reduction in dynamic binding capacity after 12.5 h 0.1 M NaOH incubation. The NaOH stability was 98.9%.

### EXAMPLE 6. Ligand detection in Protein A ELISA (leaching assay)

To determine low levels of leached variants in affinity chromatography is important for obtaining reliable results. Protein A ELISA Kits for the detection of native and recombinant Protein A (Repligen, Cat. No. 9000-1) were used for leaching assays according to manufacturer's instructions, except using 0.1 % PBST as dilution buffer. Samples: Eluted fraction of Praesto85_213108 (dimer of 209621 immobilized to Praesto 85). Internal control: protein spiking: 1 ng/ml of purified 209621 (dimer; CID213108). The leaching of the affinity ligand was 14.2 ng/ml. The dimer of 209621 showed good detectability in PBST buffer, comparable to rProtein A standard.

### EXAMPLE 7. Purification of CFH from AIC runs - Analytics

Eluted fraction of performed AIC runs were pooled and further purification was performed by size exclusion chromatography using a Superdex 200 26/600 column (GE Healthcare; buffer: PBS, pH 7.3) using an ÄKTA avant system (GE Healthcare) resulting in 40 mg purified CFH protein. Purified CFH was analyzed for homogeneity using size exclusion chromatography (SE-HPLC). Analyze has been performed on a Dionex HPLC system and a Superdex75 increase 5/150 GL (GE Healthcare). Binding to immobilized affinity ligand 209621 (dimer) was analyzed by SPR measurements according to **Example 3.** Target integrity was confirmed after purification, as shown in **FIGURE 4****.**

## Claims

1. A binding protein for complement factor H (CFH) comprising an amino acid sequence with at least 80 % sequence identity to any one selected from the group of SEQ ID NOs: 1-25 wherein the binding protein has a binding affinity of less than 1 µM for CFH.

2. A binding protein for CFH according to claim 1 wherein the CFH binding protein has a binding affinity of less than 500 nM for CFH.

3. The binding protein for CFH according to claims 1-2, wherein 2, 3, 4, 5, or 6 CFH binding proteins are linked to each other.

4. The binding protein for CFH according to claim 3, wherein the binding protein is a homo-multimer or a hetero-multimer.

5. The binding protein for CFH according to claims 1-4 wherein the binding protein is comprising additionally at least one further molecule, preferably selected from the group of (a) non-Immunoglobulin (Ig)-binding protein, or (b) a diagnostically active moiety, optionally selected from a radionuclide, fluorescent protein, photosensitizer, dye, or enzyme, or any combination of the above.

6. The binding protein for CFH according to any one of claims 1-5 for use in in the diagnosis of diseases related to CFH.

7. The binding protein for CFH according to any one of claims 1-6 for use in technical applications such as affinity purification of CFH or a protein comprising CFH.

8. An affinity separation matrix comprising a binding protein for CFH according to any one of claims 1-7.

9. Use of the binding protein for CFH according to any one of claims 1-7, or the affinity separation matrix according to claim 8 for affinity purification of CFH or a protein comprising CFH.

10. A process of manufacturing CFH comprising at least one chromatographic step employing an affinity chromatography matrix having an affinity for specifically binding CFH wherein the binding protein for CFH according to any one of claims 1-7 is coupled to said affinity chromatography matrix.

11. A method of affinity purification of CFH, the method comprising: (a) providing a liquid that contains a CFH; (b) providing an affinity separation matrix comprising at least one binding protein for CFH according to any one of claims 1-7 coupled to said affinity separation matrix; (c) contacting said affinity separation matrix with the liquid under conditions that permit binding of the at least one binding protein for CFH according to any one of claims 1-7; and (d) eluting said CFH from said affinity purification matrix.

12. Use of the binding protein for CFH according to claims 1-7, in methods to determine the presence of CFH.

13. A method of analyzing the presence of CFH in liquid samples, the method comprising the following steps:
(i) providing a liquid that contains CFH,
(ii) providing the binding protein for CFH according to claims 1-7,
(iii) contacting the liquid of (i) with the binding protein for CFH according to claims 1-7 under conditions that permit binding of the binding protein to the CFH,
(iv) isolating the complex of CFH according to claims 1-7, and
(v) determining the amount of the binding protein for CFH according to claims 1-7 in the liquid of (i).

14. A polynucleotide encoding the binding protein according to any one of claims 1-7.
